(19) Europäisches Patentamt
European Patent Office
Office européen des brevets

(11) Publication number: 0 149 255
B1

(12) # EUROPEAN PATENT SPECIFICATION

(45) Date of publication of patent specification: 06.05.87

(51) Int. Cl.$^4$: **C 07 C 29/15**, C 07 C 31/04, C 07 C 31/08, C 07 C 31/10, C 07 C 31/12

(21) Application number: 84116467.6

(22) Date of filing: 28.12.84

(54) **Process for producing alcohols from carbon monoxide and hydrogen using an alkali-molybdenum sulfide catalyst.**

(30) Priority: 30.12.83 US 567243

(43) Date of publication of application: 24.07.85 Bulletin 85/30

(45) Publication of the grant of the patent: 06.05.87 Bulletin 87/19

(84) Designated Contracting States: BE DE FR GB IT NL SE

(56) References cited:
GB-A- 469 959
US-A-1 741 307

(73) Proprietor: UNION CARBIDE CORPORATION
39 Old Ridgebury Road
Danbury Connecticut 06817 (US)

(72) Inventor: Kinkade, Nancy Ellen
314 5th Avenue
St. Albans West Virginia 25177 (US)

(74) Representative: Wuesthoff, Franz, Dr.-Ing. et al
Patentanwälte Wuesthoff -v. Pechmann-Behrens-Goetz Schweigerstrasse 2
D-8000 München 90 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 149 255 B1

Courier Press, Leamington Spa, England.

**Description**

Field of the invention

This invention relates to a catalytic process for producing alcohols directly from carbon monoxide and hydrogen; and more particularly and in a preferred embodiment to the use of an alkali-containing molybdenum sulfide heterogeneous catalyst for selectively converting carbon monoxide and hydrogen to alcohols.

Description of the prior art

It is known that carbon monoxide and hydrogen may be reacted over a suitable catalyst to produce hydrocarbons and oxygenated compounds (such as aldehydes and alcohols) containing one or more carbon atoms. Heterogeneous catalysts are known for producing alcohols. For example, U.S.—A—3,326,956 describes a process for producing oxygenated hydrocarbons, especially methyl alcohol, which comprises contacting, at elevated temperatures and pressures, a mixture of hydrogen, carbon monoxide and carbon dioxide with a solid catalyst comprising the product of partly reducing the mixed oxides of copper, zinc and chromium.

U.S.—A—4,122,110 discloses a process for manufacturing alcohols, particularly linear saturated primary alcohols, by the reaction of carbon monoxide (optionally with carbon dioxide being present also) and hydrogen in the presence of a catalyst containing four essential elements: copper, cobalt, a third metal selected from chromium, iron, vanadium and manganese, and a fourth metal which is an alkali metal; and optionally zinc. U.S.—A—4,291,126 discloses a related catalyst containing 20—60% copper, 5—50% cobalt, 5—30% of a metal selected from chromium, iron, vanadium and manganese, 5—40% of a rare earth metal, 0.1—5% of an alkali metal or alkaline earth metal and, optionally, zinc and/or a noble Group VIII metal and/or a binder.

U.S.—A—4,199,520 discloses a rhodium catalyst which is useful for the manufacture of polyhydric alcohols, particularly ethylene glycol, from synthesis gas (i.e., a mixture of predominantly carbon monoxide and hydrogen) and which comprises a rhodium carbonyl sulfur cluster compound, the anion of which may be represented by the following empirical formula $[Rh_{17}(S)_2(CO)_{32}]^{-3}$ and the cation of which includes alkali metals.

U.S.—A—4,377,643 discloses a catalytic process for converting synthesis gas to alcohols and alkanes comprising contacting carbon monoxide and hydrogen with a catalyst of the formula

$$A_aRu_bCu_cM_dN_zO_x$$

wherein A is an alkali metal, M is Rh, Ir, Pd, Pt or mixtures thereof, a is about 0.002 to about 0.5, b is about 0.5 to about 3, c is about 0.5 to about 3, d is about 0.05 to about 0.5, z is a level of 0 to about 1 weight % and x is the number of oxygens needed to fulfill the valence requirements of the other elements.

U.S.—A—4,235,798 discloses a process for selectively preparing a mixture of two-carbon atom oxygenated hydrocarbons (i.e., acetic acid, ethyl alcohol and acetaldehyde) by contacting hydrogen and carbon monoxide with a solid catalyst comprising rhodium in combination with one or more alkali metals under reaction conditions to favor the formation of such products.

U.S.—A—4,235,801 discloses a process for producing ethyl alcohol from synthesis gas employing a catalyst essentially comprising rhodium and iron.

Molybdenum-based catalysts have also been used to catalyze a variety of reactions such as desulfurization, denitrofication and hydrogenation reactions. For example, U.S.—A—2,490,488 discloses the use of a molybdenum sulfide catalyst promoted with "minor proportions" of an alkali metal oxide, hydroxide or carbonate to produce liquid hydrocarbons and organic oxygen-containing compounds from carbon monoxide and hydrogen. The preferred amount of the alkali promoter, according to the patent, is about 0.5 to 5 weight percent based on the weight of molybdenum sulfide, or 2—20 mole percent when the promoter is potassium hydroxide. Carbon monoxide and hydrogen are said to be converted to normally liquid hydrocarbons and unspecified organic oxygen-containing compounds utilizing such catalyst.

U.S.—A—4,199,522 discloses a process for the production of $C_2$—$C_4$ olefinic hydrocarbons from synthesis gas using a catalyst consisting essentially of 1—95% by weight of at least one material selected from the group consisting of the sulfide, oxide and metal of molybdenum, tungsten, rhenium, ruthenium, nickel, palladium, rhodium, osmium, iridium and platinum, and 0.05—50% by weight of at least one material selected from the group consisting of the hydroxide, oxide or salt of Li, Na, K, Rb, Cs, Mg, Ca, Sr, Ba and Th. U.S.—A—4,151,190 discloses a similar catalyst. There is no disclosure in either patent that such a catalyst produces oxygenated products.

A number of prior art references disclose so-called oxo processes which include the hydrogenation of aldehydes to alcohols in the presence of supported and unsupported molybdenum sulfide catalysts at elevated temperatures and pressures. The starting aldehydes are produced in the first step of the oxo process by carbonylating an olefin by reaction with carbon monoxide and hydrogen. These references include FR—A—1,315,275 and U.S.—A—2,709,714, 2,813,911, 2,976,254, and 3,118,954.

J. F. Shultz, et al., Bureau of Mines Report of Investigations, No. 6974, U.S. Dep't of the Interior (1967) report that molybdenum oxide and sulfide, used as methanation catalysts, produce up to 20% of the

products as two to four-carbon atom hydrocarbons. However, there is no disclosure in this article concerning alcohols or any other oxygenated products from such systems.

From the non-prepublished older EP—A1—0 119 609 is known a catalytic process for producing mixed alcohols from hydrogen and carbon monoxide using a catalyst containing molybdenum sulfide and a promoter comprising an alkali or alkaline earth metal in free or combined form, and optionally a support. The mole ratio of hydrogen to carbon monoxide is from about 0.7 to about 3. The reaction temperature is in the range of about 240 to about 325°C and the gas hourly space velocity from about 300 to 5000 $h^{-1}$. The promoter may be present as metal, oxide, hydroxide, sulfide or as a salt of the alkali or alkaline earth metals preferably as carbonate.

In addition to the foregoing the present inventor is aware of an unpublished study, by a colleague, of catalysis by molybdenum sulfide. During that study, he observed that alcohols may be produced from carbon monoxide and hydrogen using a molybdenum sulfide catalyst containing small amounts (i.e., up to about 0.30 mole of alkali per mole of molybdenum) of an alkali such as potassium hydroxide. One experimental run (at 250°C, 400 psig, a $CO/H_2$ molar ratio of 1/3, a GHSV of 3000 $h^{-1}$ and a potassium loading of about 0.15—0.18 mole per mole of molybdenum) produced the following results:

| Selectivities (%) |
|---|
| methyl alcohol=46 |
| ethyl alcohol=26 |
| n-propyl alcohol=1 |
| methane=27 |

Rate of total alcohol production=1.8 pounds per cubic foot of catalyst per hour.

In another experimental run, under the same conditions and employing a molybdenum sulfide/KOH catalyst prepared to contain about 0.30 mole potassium per mole molybdenum, the following results were obtained:

| Selectivities (%) |
|---|
| methyl alcohol=35 |
| ethyl alcohol=19 |
| n-propyl alcohol=9 |
| methane=37 |

Rate of total alcohol production=1.9 pounds/$ft^3$ of catalyst/hour.

## Summary of the invention

It has now been found that mixtures of alcohols may be produced at high selectivities and at high rates by reacting carbon monoxide and hydrogen over a heterogeneous catalyst consisting essentially of molybdenum sulfide and a significant amount of a compound of an alkali metal. In summary therefore, the process of the present invention comprises reacting carbon monoxide and hydrogen in the vapor phase, in the presence of a heterogeneous catalyst consisting essentially of molybdenum sulfide and 0.60 to 2.20 mole, per mole of molybdenum, of an alkali metal compound or mixtures thereof at a gas hourly space velocity of 6000 to 24 000 $h^{-1}$, to selectively produce mixtures of linear, $C_1$—$C_5$ alcohols at high rates. The alkali metal compound is selected from hydroxides, acetates, tartrates, citrates and sulfates of cesium, rubidium and/or potassium. Depending upon the amount of alkali metal compound in the catalyst, the reaction conditions of gas hourly space velocity, temperature and pressure may be varied to obtain the product alcohols at high selectivities and high rates.

## Description of the preferred embodiments

The process of the present invention converts carbon monoxide and hydrogen primarily to mixtures of linear, primary alcohols (i.e., substantially all primary alcohols with no carbon branching), containing principally one to five carbon atoms. Produced as by-products may be some longer-chain alcohols (i.e. $C_6$- and higher alcohols), secondary and/or branched-chain alcohols, saturated and unsaturated hydrocarbons and small amounts of aldehydes, ketones and esters. Compared to known processes for preparing such linear alcohols, the process of the present invention offers some advantages. For example, the catalyst used in the present invention is a relatively simple, easily-prepared system. Another important advantage

is the high alcohol yields obtained by the present invention. Most previously-known catalysts produce large quantities of hydrocarbons or hydrocarbons and very little alcohols. A further advantage of the process of the invention is the ability to favor the production of alcohols of longer or shorter chain lengths by careful regulation of reaction conditions. As will be seen from the following description, by suitable adjustments of certain reaction conditions (e.g., GHSV, pressure, temperatures, the particular alkali metal and alkali-loading) alcohols of different chain-lengths may be produced. While some of the prior art provides one or more of these advantages, only the present invention demonstrates all of these advantageous properties.

More specifically, the process of the present invention is useful for the selective production of mixtures of substantially linear alcohols having from 1 to 5 carbon atoms, such as methyl alcohol, ethyl alcohol, n-propyl alcohol, n-butyl alcohol, and n-pentyl alcohol. The process also produces minor proportions of secondary and branched alcohols, such as isopropyl alcohol, isobutyl alcohol, sec-butyl alcohol and iso-pentyl alcohol. However, the process is most useful in the production of $C_1$—$C_5$-normal alcohols. In fact, it has been observed that substantially all of the alcohol products of the process of the invention are normal alcohols. Normally, a mixture of alcohols is produced but by using particular catalysts and by suitable adjustments in the reaction conditions, as described below, the production of particular higher or lower alcohols may be favored. For example, under conditions which favor the higher alcohols, it is expected that the $C_5$-alcohols would be produced along with $C_4$-(and lower) alcohols.

It has also been found that when an olefin is fed to the reaction with the synthesis gas, the olefin is converted to an alcohol containing one more carbon atom than the starting olefin (subject of the EP—A—149 256).

It is desirable to control the reaction conditions to minimize formation of by-products. Typically, the most prevalent by-products are hydrocarbons (both saturated and unsaturated) although the present invention generally provides a highly selective route to alcohols over hydrocarbons. Preferably, hydrocarbon by-products are produced in amounts, relative to total alcohol production, of less than about 20 mole %. Specifically, higher gas hourly space velocities (GHSV) favor alcohol production over hydrocarbons. Other possible by-products may include small amounts of other oxygenated products such as $C_6$- and higher alcohols, secondary and/or branched-chain alcohols, and aldehydes, ketones and esters. Such oxygenated by-products are generally produced in amounts, relative to the total alcohol production, of less than about 10 mole %, optimally less than 2—3 mole %.

The reaction of the present invention is conducted in the vapor phase by reacting carbon monoxide and hydrogen in the presence of the solid, heterogeneous molybdenum sulfide-alkali metal catalyst. The desired mixture of $C_1$—$C_5$-linear, primary alcohols may be separated and recovered from the gaseous reaction products and from each other by any suitable technique known to those skilled in the art. In addition, any conventional equipment may be employed in the process of the invention, with suitable regard for the reactants, conditions of reaction and products.

The carbon monoxide and hydrogen reactants may conveniently be derived from so-called synthesis gas which is primarily a mixture of carbon monoxide and hydrogen, although it may typically also contain a very small amount of sulfur compounds as well as small amounts of carbon dioxide, and nitrogen and other inert gases, depending on its source. Synthesis gas is produced commercially, for example, as a product of the partial combustion of coal, natural gas, petroleum bottoms or other carbonaceous materials. A specific method of synthesis gas derivation is the heating of coke in the presence of air and then steam. The molar ratio of carbon monoxide to hydrogen ($CO:H_2$) in synthesis gas may vary widely from 1:10 to 10:1; for purposes of the present invention, the preferred molar ratio $CO:H_2$ is from 2:1 to 1:4. Of course, rather than employ synthesis gas, which is the preferred reactant feed, it is also possible to use as a feed gas any gas containing predominantly carbon monoxide and hydrogen within the foregoing ratios.

Regardless of the source of the carbon monoxide and hydrogen reactants, it is expected that the gas feed to the reaction may contain small amounts of sulfur compounds without deactivating substantially the molybdenum sulfide-alkali metal catalyst.

The catalyst consists essentially of molybdenum sulfide and a member selected from the group consisting of an alkali metal compound and mixtures thereof. The precise catalytic specie or species are not known with certainty. It is possible that operating temperatures, pressures, and catalyst preparation methods as well as the feed gases, may have an effect on the structure or activity of the catalyst. The precise effects are not readily ascertainable and are difficult to measure. For this reason, it is not possible to describe with reasonable certainty the structure of the active catalytic specie or species. However, it is known that when the molybdenum sulfide precursor of such catalysts is prepared by methods described hereinbelow, it exhibits an x-ray diffraction pattern characteristic of crystalline molybdenum disulfide. Since x-ray diffraction only characterizes bulk crystalline phases and it is very likely that the catalyst surface contains some oxygen in addition to molybdenum and sulfur, possibly as oxythiomolybdates, nevertheless, for ease of description, the molybdenum will be described herein sometimes as the sulfide. Subsequently, the alkali metal compound is added to that molybdenum sulfide to provide the highly selective catalyst of the invention. However, the form of the alkali metal in the active catalytic system may not be the same as the alkali metal compound that was introduced to the molybdenum sulfide. For purposes of the present invention, it is sufficient if the alkali metal compound is added as described herein, regardless of the particular form it may take in the active catalyst. The alkali metal compound may be added

to the molybdenum sulfide by the so-called incipient wetness technique, known to those skilled in the art, and described hereinbelow, or by any other method known to those skilled in the art such as ion exchange or precipitation. The resulting catalyst is said to consist essentially of molybdenum sulfide and a member selected from the group consisting of an alkali metal compound and mixtures thereof.

The catalyst may be prepared by conventional means, such as by adding an alkali metal compound to a sulfur-containing molybdenum compound such as $MoS_3$, $MoS_2$, ammonium oxythiomolybdate or ammonium polythiomolybdate (which compounds may be obtained commercially or prepared from a variety of molybdenum compounds) by, for example, impregnation with a solution of the alkali metal compound (i.e., an incipient wetness technique), co-precipitation, by grinding and calcining a dry alkali metal compound with the molybdenum compound or by ion exchange. Another method of preparing the catalyst comprises sulfiding a commercially-available alkali metal molybdate or a molybdenum oxide such as $MoS_3$ or $MoO_2$, after treatment with an alkali compound. A preferred method of preparing the catalyst comprises decomposing a thiomolybdate salt to produce molybdenum sulfide (e.g., as described in U.S.—A—4,243,553 and 4,243,554) and then adding the alkali metal compound by a suitable technique, such as the incipient wetness method. This method is preferred since it produces a catalyst more active for the production of linear, primary, $C_1$—$C_5$-alcohols than catalysts prepared by the other enumerated techniques.

More specifically, in a preferred method, molybdenum sulfide may be prepared by decomposing a thiomolybdate salt, such as ammonium thiomolybdate ("ATM") or ammonium polythiomolybdate, at an elevated temperature (e.g., on the order of 300—600°C) in a gaseous atmosphere such as nitrogen or hydrogen or mixtures thereof. Other gases may be employed, such as an inert gas, and carbon monoxide.

The ammonium thiomolybdate or other salt may be prepared by known methods, such as (in the case of ammonium thiomolybdate) by bubbling hydrogen sulfide through an ammonium hydroxide solution of ammonium heptamolybdate. $(NH_4)_6Mo_7O_{24} . 4H_2O$, at elevated temperatures (e.g., 30—40°C). The alkali metal compound may be added to the molybdenum sulfide by the so-called incipient wetness technique known to those skilled in the art. This technique generally comprises adding a pore volume of solution of the alkali metal compound to the dry molybdenum sulfide, under vacuum, followed by drying at elevated temperature under an inert gas such as nitrogen. If the amount of alkali metal compound that can be added to the molybdenum sulfide is less than desired because of limited solubility of the particular alkali metal compound employed, the procedure may be repeated until the desired alkali metal compound to molybdenum ratio is obtained.

The particular alkali metal compound contained in the catalyst of the invention does have an effect upon the alcohol selectivity of that catalyst.

The solid catalyst may be employed in any convenient form depending upon the conditions of reaction and the equipment employed. For example, the catalyst, normally obtained as a powder by grinding or milling, may be made into other conventional forms such as pellets, tablets, granules, or may be supported on any suitable inert support. Similar results have been obtained with laboratory-scale fixed-bed reactor systems employing catalyst as a powder or as 0.42—0.84 mm (20—40 mesh) particles, although on a commercial scale, it probably would be desirable to use larger mesh. In the case of fluidized-bed operations, a powdered catalyst of suitable size may be appropriate. Those skilled in the art will be able to practice the invention by selecting the form and size of catalyst, based on the conditions encountered in use. If a supported catalyst is desired, any typical inert support may be employed, such as carbon, silica, alumina, titania, silica-alumina, silica-titania, magnesia, molecular sieves and zeolites, and clays.

The supported catalysts may be prepared by techniques analogous to those employed for the unsupported catalysts, such as an incipient wetness technique using ammonium hepta molybdate and potassium acetate in aqueous solution. Supported catalysts or pelletized or extruded unsupported catalysts are favored in large-scale, fixed-bed systems. Slurry systems are conveniently operated with finer catalyst particles. The use and selection of supported or unsupported catalyst systems to suit various reactor systems is readily understood by those skilled in the art.

To produce the desired mixtures of $C_1$—$C_5$, linear, primary alcohols, the carbon monoxide and hydrogen feed are reacted in the vapor phase in suitable equipment, in the presence of catalyst, under suitable conditions of gas hourly space velocity (GHSV), temperature and pressure. The reaction can be directed to higher or lower product alcohols by suitable control of the alkali content of the catalyst and the reaction conditions.

The GHSV of the feed gas is defined as the volume of gas fed per volume of catalyst charge per hour (expressed as reciprocal hours, $h^{-1}$) and it has been unexpectedly found that high GHSV's result in high selectivities to the desired alcohols. For purposes of the present invention, the GHSV should be at least 6000 $h^{-1}$, most preferably at least about 12,000 $h^{-1}$. With lower GHSV's, hydrocarbon selectivity increases at the expense of alcohol selectivity. With GHSV's much higher than 12000 $h^{-1}$, alcohol selectivity is not substantially changed and while the rate of alcohol production does increase with higher GHSV's the total conversion to product decreases slightly.

The temperature at which the process of the invention may be performed is not particularly critical and may be varied depending on the results desired. As a general rule, the temperature of the process may be from 250°C to 400°C, and a temperature of from 275°C to 350°C is preferred. As can be seen from the examples which follow, use of higher process temperatures tends to favor production of longer-chain

alcohols over shorter-chain alcohols. However, when higher temperatures are used, a larger proportion of hydrocarbons is also produced. Therefore, depending on the particular catalyst, the reaction conditions may be varied to obtain the desired results.

The pressure of the reaction is not critical and may be from about atmospheric to about 138 bar (2.000 psig), depending on the equipment used and results desired. Generally, increasing pressure tends to favor alcohols over hydrocarbons but also favors shorter-chain products (both alcohols and hydrocarbons) over longer-chain products.

The products $C_1$—$C_5$-alcohols have many and varied industrial uses. For example, they are useful as solvents and also as intermediates for the production of, for example, plasticizers for synthetic resins or as fuel additives.

The examples which follow are meant to further illustrate the present invention (except for the comparative Examples).

Except as noted in the examples (all of which describe work actually performed), the molybdenum sulfide catalyst was prepared by decomposing ammonium thiomolybdate (ATM) at an elevated temperature, usually 400°C, in nitrogen containing 10 volume percent hydrogen. The ammonium thiomolybdate was prepared by bubbling hydrogen sulfide through an ammonium hydroxide solution of ammonium hepta molybdate, $(NH_4)_6Mo_7O_{24} . 4H_2O$, at 30—40°C. The molybdenum sulfide prepared in this manner exhibited an x-ray diffraction pattern typical of molybdenum disulfide and surface areas of 10—60 $m^2/g$. The alkali metal compound additive was then introduced into the molybdenum sulfide by an incipient wetness technique. Specifically, the molybdenum sulfide was first evacuated for 1 hour using a rotary vacuum pump and then an aqueous solution of the alkali metal compound was introduced onto the molybdenum sulfide with a syringe. The volume of the solution added was equivalent to one pore volume of the molybdenum sulfide, i.e., the volume required to fill the void volume of the molybdenum sulfide. If excess liquid was added, the catalyst was allowed to dry under a vacuum until no liquid was present. The resulting wet material was dried under a flow of nitrogen at 85°C for 1 hour and then 110°C for 2 hours. It was then ground to a uniform powder and added to a tube at 400°C under a flow of 20 parts by volume of hydrogen and 180 parts by volume of nitrogen. After one hour at 400°C, the hydrogen flow was stopped and the material cooled to ambient temperature.

The supported catalysts employed in the examples were also prepared by the incipient wetness technique using ammonium hepta molybdate and potassium acetate in an aqueous solution. The inert supports were washed with oxalic acid before use to remove impurities by passing a mixture of oxalic acid, glycerine, and water in proportions of 1:1.5:2.5 by weight, respectively, through a bed of support material (length/diameter ratio of about 20 to 25) contained within a glass tube which drained through a stopcock at its base. The contents of the tube were maintained at about 90°C by means of resistance heating wire wrapped around the exterior of the tube. About 2.5 volumes of oxalic acid solution were used to wash 1 volume of 0.42—0.84 mm (8—20 mesh) support material over a 3-hour period. The material was then washed with about 6 volumes of distilled water at 90°C over a period of about 4 hours and then dried at 350°C for about 4 hours.

The reactor used in the examples was a continuous feed, U-shaped, stainless steel, tubular reactor with no recycle. Gas samples were routinely analyzed by conventional gas chromatographic techniques. Product identifications were initially confirmed by the use of a mass spectrometer. The catalyst was loaded into the stainless steel U-tube with 0.5 mm quartz beads above and below the catalyst bed. If an unsupported catalyst was used, it was prepared as powder and then mixed with an equal volume of 0.5 mm quartz beads. Supported catalysts were loaded as 0.42—0.84 mm (20—40 mesh) particles. Once installed, the reactor was pressure tested and then flushed several times with nitrogen before the reactant gas mixture (carbon monoxide and hydrogen) flow was started. After the desired flow and pressure were established, the reactor was heated to reaction temperature in a fluidized sand bath. A gas sample was analyzed 1 hour after the fluidized sand bath reached the reaction temperature and every hour thereafter until the end of the run. Modifications to the reaction conditions were made immediately after a gas sample was injected into the chromatograph and the first sample at the new conditions was analyzed 1 hour later. The reactor was cooled to room temperature after each run with a slow flow of nitrogen passing through the catalyst. The data in the examples represents an average of several analyses.

Conversion was determined as the percent of carbon monoxide fed to the reactor converted to product; and selectivity to a particular product is stated as the number of moles of carbon in that particular product as a percentage of the total number of moles of carbon in all products. A product is defined as any compound which is derived from synthesis gas other than $CO_2$ or $H_2O$.

Examples 1, 2 and Comparative Examples A—C

A series of catalysts were prepared by the method described above, all containing 0.616 mole of different alkali metal or alkaline earth metal compounds per mole of molybdenum. These catalysts were charged to a tubular reactor as described above and carbon monoxide and hydrogen were reacted under the following reaction conditions:

| | |
|---|---|
| temperature (°C) | 300 |
| pressure (psig) bar | (400) 27.6 |
| GHSV ($h^{-1}$) | 12,000 |
| $H_2/CO$ (molar): | 1 |

The results are shown in Table I below.

TABLE I

| Example | Alkali compound | Conversion % | Selectivity, % MeOH | EtOH | PrOH | BuOH | H.C. | Alcohol rate (lb/CF/hr) kg/m³ h |
|---|---|---|---|---|---|---|---|---|
| 1 | rubidium acetate | 2.3 | 36 | 23 | 6 | 1 | 31 | (6.3) 100.8 |
| 2 | cesium acetate | 4.8 | 47 | 27 | 5 | 1 | 18 | 16.0 256 |
| Comparative Examples | | | | | | | | |
| A | magnesium acetate | 3.1 | — | — | — | — | 100 | — |
| B | calcium acetate | 0.8 | 5 | 1 | — | — | 94 | (1) 16 |
| C | barium hydroxide | 0.4 | 26 | 11 | 4 | — | 59 | 0.7 11.2 |

Notes:
MeOH=Methyl alcohol
EtOH=Ethanol
PrOH=Total propanol
BuOH=Total butanol
H.C.=Total hydrocarbons
lb/CF/hr=Total pounds of alcohols produced per cubic foot of catalyst per hour
kg/$m^3$ h.

Examples 3—6

Example 1 was repeated except that the anion of a potassium compound and total potassium in the catalyst system were varied. The results are shown in Table II below. The symbols have the same meaning as those used in Table I.

TABLE II

| Example | Anion | Molar ratio of potassium to molybdenum | Conversion % | Selectivity, % | | | | | Alcohol rate (lb/CF/hr) $kg/m^3$ h |
|---|---|---|---|---|---|---|---|---|---|
| | | | | MeOH | EtOH | PrOH | BuOH | H.C. | |
| 3 | hydroxide | 0.20 | 6.5 | 16.8 | 16.8 | 12.4 | 2.3 | 51 | (11.6) 185.6 |
| 4 | hydroxide | 0.60 | 4.4 | 35.5 | 36.6 | 5.6 | 0.6 | 19 | (13.8) 220.8 |
| 5 | acetate | 0.60 | 1.7 | 39.5 | 29.7 | 8.0 | 2.2 | 17.9 | (5.6) 89.6 |
| 6 | acetate | 2.20 | 2.6 | 43.6 | 27.2 | 8.7 | 0.8 | 14.9 | (9.0) 144 |

Examples 7—10

These examples illustrate the wide variety of anions which may be used as part of the alkali metal compound which forms a part of the catalyst used in the process of the present invention. Examples 7 and 8 illustrate the use of potassium tartrate, while examples 9 and 10 show the use of potassium citrate. Both of these compounds give good rates and selectivities, but the citrates are preferred.

These examples also serve to demonstrate the effect of higher gas hourly space velocities in the process of the present invention. From these examples, it can be seen that an increase in the gas hourly space velocity from 12,000 $h^{-1}$ to 24,000 $h^{-1}$ causes a slight decrease in total conversion but significantly increases the alcohol production rate and the selectivity towards alcohols.

The reaction conditions and results of these examples are set forth in Table III below.

TABLE III

| Example | Anion | Molar ratio of potassium to molybdenum | Conversion % | Selectivity (%) | | | | | Alcohol rate (lb/CF/hr) kg/m$^3$ h |
|---|---|---|---|---|---|---|---|---|---|
| | | | | MeOH | EtOH | PrOH | BuOH | H.C. | |
| 7 | Tartrate[a] | 0.6 | 1.4 | 50.0 | 30.8 | 3.1 | N.D. | 15.2 | (5.3) 84.8 |
| 8 | Tartrate[b] | 0.6 | 1.3 | 58.4 | 28.8 | 5.2 | 0.6 | 6.4 | (11.5) 184 |
| 9 | Citrate[c] | 0.6 | 2.0 | 42.9 | 31.6 | 3.3 | 0.1 | 20.7 | (6.4) 102.4 |
| 10 | Citrate[d] | 0.6 | 1.7 | 54.5 | 30.4 | 6.1 | 0.7 | 7.7 | (14.0) 224 |

[a] Reaction conditions: 300°C, 27.6 bar (400 psig), $H_2$:CO=1:1, GHSV=12,000 $h^{-1}$
[b] Reaction conditions: 300°C, 27.6 bar (400 psig) $H_2$:CO=1:1, GHSV=24,000 $h^{-1}$
[c] Reaction conditions: 300°C, 27.6 bar (400 psig) $H_2$:CO=1:1, GHSV=12,000 $h^{-1}$
[d] Reaction conditions: 300°C, 27.6 bar (400 psig) $H_2$:CO=1:1, GHSV=24,000 $h^{-1}$

Examples 11—13 and Comparative Example D

The use of various cesium compounds is demonstrated in Examples 11—13. In these examples the anion of the cesium compound and the mole ratio of cesium compound to molybdenum was varied. The catalyst system was charged to the reactor and the reaction was conducted at a pressure of 27.6 bar (400 psig), a temperature of 300°C, a GHSV of 12,000 $h^{-1}$ and a $CO/H_2$ molar ratio of 1.0. The results are set forth in Table V below. The symbols are the same as in Table I.

TABLE IV

| Example | Anion | Mole ratio of cesium to molybdenum | Conversion % | Selectivity % | | | | | Alcohol rate (lb/CF/hr) kg/m³ h |
|---|---|---|---|---|---|---|---|---|---|
| | | | | MeOH | EtOH | PrOH | BuOH | H.C. | |
| 11 | acetate | 0.33 | 4.4 | 27 | 28 | 9 | 2 | 33 | (11.4) 182.4 |
| 12 | acetate | 0.60 | 4.8 | 47 | 27 | 5 | 1 | 18 | (16) 256 |
| 13 | sulfate | 0.60 | 3.5 | 33.8 | 30.6 | 7.7 | 1.1 | 17.6 | (10.2) 163.2 |
| D | chloride | 0.60 | 2.2 | 2. | 6. | 3. | 0.1 | 88. | (1) 16 |

Examples 14—21

The molybdenum sulfide used in Examples 1—16 and 11—13 above was pretreated at 400°C in a mixture of 10 parts by volume of hydrogen and 90 parts by volume of nitrogen. When the pretreatment temperature was raised, the selectivity tends to shift toward the higher alcohols. The following Table V shows the results obtained using a catalyst system containing 0.616 mole of potassium acetate per mole of molybdenum at various molybdenum sulfide pretreatment temperatures, in a mixture of 10 parts of hydrogen and 90 parts of nitrogen. The other reaction conditions were: GHSV=12,000 $h^{-1}$; $H_2/CO$ (molar)=1.0; pressure=27.6 bar (400 psig). The symbols are the same as in Table I.

TABLE V

| Example | Pretreatment temp. °C | Reaction temp. °C | Conversion % | Selectivity (%) | | | | | Alcohol rate (lb/CF/hr) $kg/m^3$ h |
|---|---|---|---|---|---|---|---|---|---|
| | | | | MeOH | EtOH | PrOH | BuOH | H.C. | |
| 14 | 400 | 300 | 1.7 | 39.5 | 29.7 | 8.0 | 2.2 | 16.3 | (5.6) 89.6 |
| 15 | 400 | 300 | 2.1 | 44.0 | 47.0 | 8.0 | 1.0 | 15.0 | (7.1) 113.6 |
| 16 | 400 | 350 | 3.4 | 25.0 | 47.0 | 23.0 | 6.0 | 25.0 | (8.2) 131.2 |
| 17 | 400 | 350 | 3.8 | 15.4 | 23.9 | 11.6 | 2.9 | 41.7 | (7.7) 123.2 |
| 18 | 500 | 300 | 3.0 | 23.8 | 42.5 | 14.1 | 3.4 | 16.0 | (9.0) 144 |
| 19 | 500 | 344 | 5.1 | 12.4 | 32.3 | 18.5 | 4.2 | 17.6 | (11.3) 180.8 |
| 20 | 600 | 300 | 0.7 | 26.9 | 38.2 | 12.5 | 3.3 | 14.8 | (2.1) 33.6 |
| 21 | 600 | 340 | 1.7 | 20.0 | 41.0 | 27.0 | 11.0 | 26.0 | (3.5) 56 |

Examples 22—27

A catalyst system of 0.85—0.90 mole of cesium acetate per mole of molybdenum, was prepared and charged into the reactor as described above. Several runs were made at a pressure of 27.6 bar (400 psig), a GHSV of 12,000 $h^{-1}$ and a $H_2/CO$ molar ratio of 1.0. The temperature was varied to demonstrate the effect of temperature on alcohol production. Similar runs were made under the same conditions using a catalyst of molybdenum sulfide and 0.85—0.90 mole of potassium acetate per mole of molybdenum. The data are summarized in Table VI and show that higher operating temperatures favor the production of longer chain alcohols. The symbols mean the same as those used in Table I.

TABLE VI

| Example | Alkali metal | Reaction temp. °C | Selectivity % * | | Selectivity % ** | | | | Alcohol rate |
|---|---|---|---|---|---|---|---|---|---|
| | | | H.C. | Total alcohol | MeOH | EtOH | PrOH | BuOH | (lb/G/h) kg/$m^3$ h |
| 22 | Ca | 250 | 7 | 90 | 86 | 11 | 2 | 1 | (3.7) 59.2 |
| 23 | Ca | 300 | 18 | 80 | 59 | 34 | 6 | 1 | (16.0) 256 |
| 24 | Ca | 350 | 28 | 66 | 34 | 29 | 29 | 8 | (9.0) 144 |
| 25 | K | 300 | 15 | 81 | 55 | 33 | 11 | 1 | (9.0) 144 |
| 26 | K | 325 | 20 | 73 | 38 | 36 | 22 | 4 | (9.7) 155.2 |
| 27 | K | 350 | 33 | 65 | 25 | 34 | 32 | 9 | (10.9) 174.4 |

* of total converted product
** of total alcohol products.

Examples 28—32

Catalysts may be prepared by several methods other than the incipient wetness technique using molybdenum sulfide prepared from ATM, such as by sulfiding potassium molybdate *in situ*, impregnating commercially-available molybdenum disulfide, and intimately mixing dry potassium acetate and molybdenum sulfide. The results obtained when such catalysts are used in the process of the present invention are shown in Table VIII. Example 28 shows results obtained using a commercially-available potassium molybdate sample sulfided *in situ* with hydrogen disulfide at 400°C. The material contained a potassium:molybdenum molar ratio of 0.82. This catalyst did result in good selectivity to alcohols but at a lower rate than the other catalysts discussed. Examples 29 and 30 show the results for catalysts prepared by the incipient wetness technique using potassium acetate and commercially-available molybdenum disulfide with molar ratios of potassium to molybdenum of 0.60 and 2.00, respectively. These resulted in reasonable selectivity to alcohols but lower activity. Examples 31 and 32 are the results for catalysts prepared by grinding together dry potassium acetate and molybdenum sulfide prepared by the decomposition of ATM and then calcining at 400°C in a mixture of 10 parts by volume hydrogen and 90 parts by volume of nitrogen. The catalyst used in Example 44 contained a potassium:molybdenum molar ratio of 0.60 and exhibited a moderate rate of alcohols formation. Example 42 utilized a catalyst that contained a potassium:molybdenum molar ratio of 2.00 and shows a more desirable ratio of alcohol to hydrocarbon production distribution while retaining reasonable alcohol productivity. The symbols are the same as in Table I.

TABLE VII

| | | Selectivity % | | | | | |
|---|---|---|---|---|---|---|---|
| Example | Conv. % | MeOH | EtOH | PrOH | BuOH | H.C. | Alcohol rate (lb/CF/hr) kg/m³ h |
| 28 | 1.1 | 52.5 | 25.7 | 6.6 | 0.5 | 13.0 | (1.04) 16.6 |
| 29 | 0.15 | 37.9 | 25.2 | 9.1 | — | 22.1 | (0.44) 7 |
| 30 | 0.12 | 42.8 | 21.5 | 9.3 | — | 24.7 | (0.35) 5.6 |
| 31 | 2.43 | 20.0 | 10.9 | 5.7 | 2.5 | 54.0 | (3.69) 59 |
| 32 | 0.87 | 46.6 | 24.0 | 7.3 | 1.6 | 19.0 | (2.89) 46.2 |

Examples 33—37

The use of optional supports for the catalysts employed in the process of the present invention is illustrated in Examples 33—37. A synthesis gas mixture was contacted with various supported catalysts at a temperature of 300°C, a pressure of 27.6 bar (400 psig) and a GHSV of 12,000 $h^{-1}$. The supports used, the amount of support used and the resultant alcohol production are set forth in Table VIII below. This data shows that the use of a magnesium oxide support will more strongly favor the production of shorter-chain alcohols than will the use of alumina or silica as supports. The symbols are the same as in Table I.

TABLE VIII

| Example | Support | Catalyst composition[e] | | Conv. | Selectivity % | | | | | Alcohol rate |
|---|---|---|---|---|---|---|---|---|---|---|
| | | wt% Mo | wt% K | % | MeOH | EtOH | PrOH | BuOH | H.C. | (lb/CF/h) kg/m$^3$ h |
| 33 | Alumina[a] | 24 | 3.6 | 0.69 | 9.8 | 31.0 | 18.4 | 3.0 | 31.6 | (1.57) 25.1 |
| 34 | Alumina[b] | 24 | 1.9 | 0.43 | 42.7 | 31.8 | 4.5 | — | 19.9 | (1.44) 23 |
| 35 | Alumina[b] | 24 | 3.6 | 0.25 | 62.0 | 16.03 | — | — | 18.5 | (0.92) 14.7 |
| 36 | Silica[c] | 24 | 3.6 | 0.64 | 12.9 | 36.4 | 16.0 | — | 32.5 | (1.6) 25.6 |
| 37 | MgO[d] | 24 | 3.6 | 0.72 | 60.4 | 20.3 | 12.7 | 0.8 | 4.5 | (1.74) 27.8 |

[a] alumina ("SA6173") obtained from the Norton Co. and washed with oxalic acid.
[b] alumina ("SA5534") obtained from the Norton Co. and washed with oxalic acid.
[c] silica ("59") obtained from the Davison Co. and washed with oxalic acid.
[d] magnesium oxide ("5500") produced by the Norton Co.
[e] weight percentages are based on the weight of the entire catalyst including support.

**Claims**

1. A process for the selective production of mixtures of linear primary alcohols having 1 to 5 carbon atoms comprising reacting hydrogen and carbon monoxide in the vapor phase in the presence of a catalyst consisting essentially of molybdenum sulfide and from 0.60 to 2.20 mole, per mole of molybdenum, of an alkali metal compound or mixtures thereof selected from hydroxides, acetates, tartrates, citrates and sulfates of alkali metals and the alkali metal is cesium, rubidium or potassium and wherein the hydrogen and carbon monoxide gas reactants are fed to the reaction at a rate of from 6,000 to 24,000 liters of gas per liter of catalyst per hour.

2. The process of claim 1 wherein the alkali metal compound is selected from potassium, rubidium and cesium acetate, potassium tartrate or citrate, cesium sulfate and mixtures thereof.

3. The process of claims 1 to 2 wherein the molar ratio $CO:H_2$ is from 2:1 to 1:4.

4. The process of claims 1 to 3 wherein the temperature of reaction is from 250°C to 400°C.

5. The process of claims 1 to 4 wherein the molybdenum sulfide is obtained by heating ammonium thiomolybdate at elevated temperatures, preferably at a temperature of 300°C to 600°C.

6. The process of claim 2 where the alkali metal compound is potassium or cesium acetate.

**Patentansprüche**

1. Verfahren zur selektiven Bildung von Gemischen linearer primärer Alkohole mit 1 bis 5 Kohlenstoffatomen durch Umsetzung von Wasserstoff und Kohlenmonoxid in Dampfphase in Gegenwart eines Katalysators, bestehend im wesentlichen aus Molybdänsulfid und 0,60 bis 2,20 mol je mol Molybdän einer Alkaliverbindung in Form der Hydroxide, Acetate, Tartrate, Citrate und Sulfate oder deren Gemisch, wobei das Alkali Caesium, Rubidium oder Kalium ist und die gasförmigen Reaktionspartner Wasserstoff und Kohlenmonoxid mit einer Geschwindigkeit von 6 000 bis 24 000 l Gas je Liter Katalysator und Stunde der Reaktion zugeführt werden.

2. Verfahren nach Anspruch 1, worin die Alkaliverbindung Caesium-, Rubidium- oder Kaliumacetat, Kaliumtartrat oder -citrat, Caesiumsulfat oder deren Gemische ist.

3. Verfahren nach Anspruch 1 oder 2, worin das Molverhältnis $CO:H_2$ 2:1 bis 1:4 ist.

4. Verfahren nach Anspruch 1 bis 3, worin die Reaktionstemperatur 250 bis 400°C ist.

5. Verfahren nach Anspruch 1 bis 4, worin das Molybdänsulfid erhalten worden ist durch Erhitzen von Ammoniumthiomolybdat auf erhöhte Temperaturen, vorzugsweise eine Temperatur von 300 bis 600°C.

6. Verfahren nach Anspruch 2, worin die Alkaliverbindung Kalium- oder Caesiumacetat ist.

**Revendications**

1. Procédé de production sélective de mélanges d'alcools primaires linéaires ayant 1 à 5 atomes de carbone, comprenant la réaction d'hydrogène et d'oxyde de carbone en phase vapeur en présence d'un catalyseur, essentiellement constitué de sulfure de molybdène et de 0,60 à 2,20 moles, par mole de molybdène, d'un composé de métal alcalin ou de mélanges de tels composés choisis entre des hydroxydes, des acétates, des tartrates, des citrates et des sulfates de métaux alcalins, et le métal alcalin est le césium, le rubidium ou le potassium, et dans lequel l'hydrogène et l'oxyde de carbone constituant les corps réactionnels gazeux sont chargés dans la réaction à une vitesse de 6000 à 24 000 litres de gaz par litre de catalyseur par heure.

2. Procédé suivant la revendication 1, dans lequel le composé de métal alcalin est choisi entre les acétates de potassium, rubidium et césium, le tartrate ou le citrate de potassium, le sulfate de césium et leurs mélanges.

3. Procédé suivant les revendications 1 et 2, dans lequel le rapport molaire $CO:H_2$ va de 2:1 à 1:4.

4. Procédé suivant les revendications 1 à 3, dans lequel la température de réaction va de 250°C à 400°C.

5. Procédé suivant les revendications 1 à 4, dans lequel le sulfure de molybdène est obtenu par chauffage de thiomolybdate d'ammonium à des températures élevées, de préférence à une température de 300°C à 600°C.

6. Procédé suivant la revendication 2, dans lequel le composé de métal alcalin est l'acétate de potassium ou de césium.